# EUROPÄISCHE PATENTSCHRIFT

(11) **EP 0 960 878 B1**
(45) Veröffentlichungstag und Bekanntmachung des Hinweises auf die Patenterteilung: **22.03.2006**
(21) Anmeldenummer: 99109940.9
(22) Anmeldetag: 20.05.1999
(51) Int. Cl.: C07C 231/02

(54) **Verfahren zur Herstellung von Beta-Hydroxyalkylamiden**
Process for the preparation of beta-hydroxyalkylamides
Procédé pour la préparation de bêta-hydroxyalkylamides

(30) Priorität: 28.05.1998 DE 19823925
(43) Veröffentlichungstag der Anmeldung: 01.12.1999
(73) Patentinhaber: EMS-Chemie AG, 7013 Domat/Ems (CH)
(72) Erfinder: Kaplan, Andreas Dr.rer.nat., 7000 Chur (CH); Gisler, René, 7000 Chur (CH); Reich, Albert, 7014 Trin (CH)
(74) Vertreter: Pfenning, Meinig & Partner GbR

(56) Entgegenhaltungen:
- US-A- 4 076 917
- US-A- 4 493 909
- US-A- 4 727 111
- US-A- 5 101 073
- US-A- 5 646 318

## Beschreibung

Die folgende Erfindung beschreibt ein Verfahren zur Herstellung, Reinigung und Isolation von β-Hydroxyalkylamiden, die als chemische Zwischenprodukte und als chemische Vernetzer für carboxylfunktionelle Polyester und Acrylate in lösemittelhaltigen Lacken, Wasser- und Pulverlacken eingesetzt werden. Besonders bei der Verwendung in Pulverlacken werden sehr hohe Anforderungen an die Erscheinungsform der β-Hydroxyalkylamide gesetzt. Nur freifließende Pulver, d.h. nicht weiche, klebrige oder wachsartige, sind für den Einsatz in Pulverlacken geeignet.

β-Hydroxyalkylamide werden durch Aminolyse von Alkylestern mit β-Aminoalkoholen in Gegenwart von basischen Katalysatoren, wie z.B. Natronlauge oder Natriummethylat hergestellt, wobei die β-Aminoalkohole in den meisten Fällen aufgrund der Selektivität der Reaktion im Überschuß eingesetzt werden.

Bei flüssigen β-Hydroxyalkylamiden müssen die nicht umgesetzten β-Aminoalkohole aus dem Reaktionsgemisch entfernt werden, bevor sie der Anwendung zugeführt werden können. Bei festen β-Hydroxyalkylamiden erfolgt die Isolation und die Reinigung der β-Hydroxyalkylamide entweder durch Kristallisation in einem Lösemittel (J. Coat. Tech., 50(643), 49-55 (1978), US 4,076,917, US 4,727,111) oder speziell bei festen β-Hydroxyethylamiden lösemittelfrei in einem sogenannten slurry-Verfahren (US 5,101,073) direkt aus dem Reaktionsgemisch.

Bei der Kristallisation in Lösemittel werden im allgemeinen in der Wärme entweder die β-Hydroxyalkylamide zu einem Lösemittel, wie z.B. Methanol und/oder Aceton oder das Lösemittel zu den β-Hydroxyalkylamiden gegeben. Nach Abkühlung der Lösung und Kristallisation werden dann anschließend die β-Hydroxyalkylamide abfiltriert und durch Trocknen vom Lösemittel befreit. Die Ausbeute wird durch die Löslichkeit in dem verwendeten Lösemittel verringert. Zudem kann der im Reaktionsgemisch verbleibende Katalysator zu unerwünschten Nebenreaktionen führen, wie z.B. zu Diacetonalkolhol bei der Verwendung von Aceton als Lösemittel, wodurch auch Verluste bei der Rückgewinnung des verwendeten Lösemittels auftreten. Weiterhin zeigt sich, daß nicht umgesetzte β-Aminoalkohole bei der Kristallisation als unerwünschte Verunreinigungen mit ausfallen und zudem wirken β-Aminoalkohole als Lösevermittler, die die Kristallisation negativ beeinflussen. Dadurch wird die Ausbeute an β-Hydroxyalkylamiden nochmals verringert.

Speziell die Herstellung von festen β-Hydroxy-ethylamiden kann lösemittelfrei in der Schmelze in einem sogenannten slurry-Verfahren erfolgen. Das slurry-Verfahren (US 5,101,073) beruht darauf, daß die bei der Herstellung von β-Hydroxyethylamiden ablaufenden Gleichgewichtsreaktionen in Richtung des gewünschten Endproduktes β-Hydroxyethylamid dadurch verschoben werden, daß durch Tempern in einem bestimmten Temperaturbereich das gewünschte β-Hydroxyethylamid aus der Schmelze ausfällt und dadurch die Schmelze kristallisiert. Bei Substanzen, bei denen das gewünschte β-Hydroxyalkylamid nicht aus der Schmelze ausfällt, z.B. bei β-Hydroxypropylamiden, versagt das slurry-Verfahren. Zudem ist das slurry-Verfahren auf den Einsatz von equimolaren Mengen an Alkylestern und β-Hydroxy-ethylamiden beschränkt. Beim slurry process entstehen bei einem äquimolekularen Einsatz von Dialkylestern mit β-Hydroxyethylaminen in Gegenwart von basischen Katalysatoren, wie z.B. Natronlauge oder Natriummethylat, neben den gewünschten monomeren β-Hydroxyethylamiden (I) als Nebenprodukte auch sogenannte Dimere (II) und Esteramide (III). Ferner enthält das Reaktionsprodukt noch β-Hydroxyethylamin. 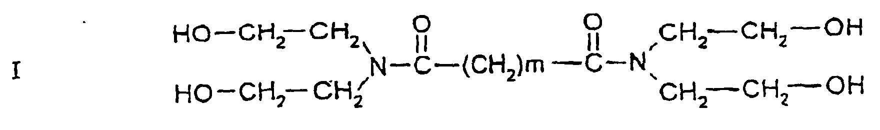 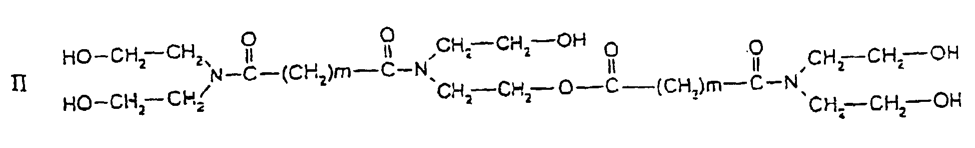 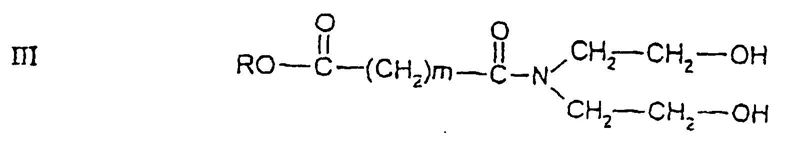 wobei m=0 bis 10 und R eine C1-C5-Niederalkylgruppe ist.

Ein wichtiger Verfahrensschritt bei der Herstellung von β-Hydroxyalkylamiden durch Aminolyse von Alkylestern mit β-Aminoalkoholen in Gegenwart von basischen Katalysatoren wie z.B. Natronlauge oder Natriummethylat, ist die Entfernung von nicht umgesetzten β-Aminoalkoholen aus dem Reaktionsgemisch.

Bekannt aus der oben genannten Literatur ist bisher die Abtrennung des überschüssigen β-Aminoalkohols durch Lösen in einem geeigneten Lösemittel, wie z.B. Methanol und anschließende Entfernung des β-Aminoalkohols mit Hilfe eines Ionenaustauschers. Danach wird durch Abdestillieren des Lösemittels das gewünschte β-Hydroxyalkylamid enthalten. Allerdings ist diese Methode nur für den Laboratoriumsmaßstab geeignet. Die Abtrennung von Nebenprodukten mittels Ionenaustauscher eignet sich nämlich nur für die Abtrennung von geringen Mengen an Nebenprodukten. Bei einem hohen Anteil an Nebenprodukten, d.h. bei Verwendung eines großen Überschusses von β-Aminoalkohol bei der Reaktion ist ein solches Verfahren technisch sehr aufwendig und unwirtschaftlich.

Aus der US 5,646,318 ist ein weitens Verfahren zur herstellung von hydroxyalkylamino bekannt.

Ausgehend von der US 5,101,073 ist es die Aufgabe der vorliegenden Erfindung ein verbessertes Verfahren in bezug auf die Reinheit, Ausbeutung und Variabilität der herzustellenden β-Hydroxyalkylamide anzugeben, sowie deren Verwendung aufzuzeigen. Die Aufgabe wird durch die kennzeichnenden Merkmale des Anspruches 1 gelöst. Die Unteransprüche zeigen vorteilhafte Weiterbildungen auf.

Überraschenderweise wurde nun gefunden, daß bei der Herstellung von β-Hydroxyalkylamiden durch Aminolyse von Alkylestern mit einem Überschuß an β-Aminoalkoholen in Gegenwart von basischen Katalysatoren, wie z.B. Natronlauge oder Natriummethylat, nicht umgesetzte β-Aminoalkohole direkt aus dem Reaktionsgemisch durch Destillation entfernt werden können, nachdem zuvor der basische Katalysator zerstört wurde, z.B. durch eine anorgansiche oder organische Säure, wie z.B. Salzsäure oder Essigsäure. Ein Überschuß an β-Aminodialkohol bei der Herstellung der β-Hydroxyalkylamiden erhöht die Selektivität der Reaktion in Richtung des gewünschten monomeren Endproduktes. Dadurch wird ein noch reineres Produkt erhalten, dies zeigt sich bei festen β-Hydroxyalkylamiden auch im Anstieg des Schmelzpunktes und in einem verbesserten Kristallisationsverhalten. Die so hergestellten β-Hydroxyalkylamide zeichnen sich durch eine sehr hohe Reinheit aus. Für die Entfernung des nicht umgesetzten Aminalkohols eignen sich neben der klassischen Destillation bei Normaldruck oder unter Vakuum besonders die Kurzweg-, die Dünnschicht und die Fallfilmdestillation, da diese Destillationsverfahren besonders produkteschonend sind, wodurch unerwünschte Nebenreaktionen durch zu lange Temperaturbelastung vermieden werden können. Ohne Zerstörung des Katalysators können bei der Destillation unerwünschte Nebenreaktionen stattfinden, es können sich z.B. die oben beschriebenen Dimeren (III) wieder bilden, die sich negativ auf die Produkteeigenschaften der β-Hydroxyalkylamide, wie z.B. bei festen β-Hydroxyalkylamide auf den Schmelzpunkt oder das Kristallisationsverhalten, auswirken. Ein weiterer Vorteil der Zerstörung des Katalysators liegt darin, daß die überschüssigen β-Aminoalkohole in reiner Form ohne Nebenprodukte zurückgewonnen werden können und für weitere Umsetzungen genutzt werden können. Flüssige β-Hydroxyalkylamide können ohne weiteren Aufarbeitungsschritt direkt verwendet werden. Die Isolation des Endproduktes bei festen β-Hydroxyalkylamiden erfolgt entweder direkt durch Kristallisation des Reaktionsproduktes oder durch Kristallisation aus einem Lösemittel, da die oben genannten Nachteile bei der Kristallisation nach der Zerstörung des Katalysators und der Entfernung von nicht umgesetztem β-Aminoalkohol nicht mehr auftreten. Das Verfahren kann batchweise und/oder kontinuierlich durchgeführt werden.

Beansprucht wird ein Verfahren zur Herstellung von β-Hydroxyalkylamiden (IV) 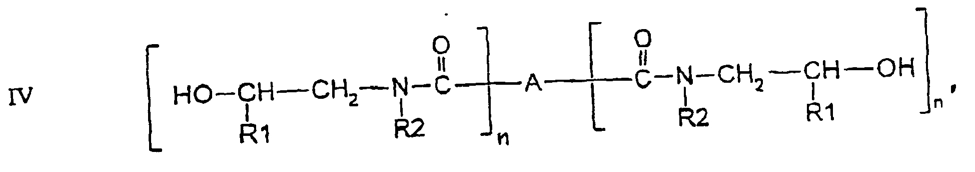 wobei A eine chemische Bindung oder eine mehrwertige organische Gruppe ist oder, wenn n'=0 ist kann A Wasserstoff oder eine einwertige organische Gruppe sein, wobei die ein- oder mehrwertige organische Gruppe ausgewählt ist aus gesättigten oder ungesättigten (C1-C60) Alkyl-, Cycloalkyl-, Aryl-, carboxyalkenyl-, Alkoxycarbonylalkenyl- oder Trialkylenaminogruppen, wobei bei den drei letzt genannten Gruppen niedere Alkenylgruppen, d.h. Alkenylgruppen mit 1 bis 20 C-Atomen bevorzugt sind. R1 ist Wasserstoff oder eine C1-C5-Alkylgruppe, R2 ist Wasserstoff, eine C1-C5-Alkylgruppe oder: 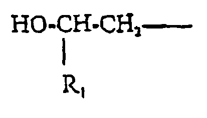 n ist eine ganze Zahl mit einem Wert von 1 bis 10 und n' ist eine ganze Zahl mit einem Wert von 0 bis 2. In Abwesenheit von Lösemitteln werden Alkylester mit β-Aminoalkoholen in Gegenwart von basischen Katalysatoren umgesetzt, wobei zur Verbesserung der Selektivität das Verhältnis Esteräquivalent zu Aminäquivalent 1 : 1,05 bis 6 beträgt. Erfindungsgemäß wird somit der Aminalkohol in einen Überschuß von 5 bis 600 % umgesetzt. Besonders bevorzugt ist ein Verhältnis von 1 : 1,1 bis 1 : 2. Der bei der Reaktion entstehende Alkohol wird bei geeigneten Temperaturen eventuell unter vermindertem Druck aus dem Reaktionsgemisch entfernt. Nach Zerstörung des basischen Katalysators, z.B. durch eine geeignete anorganische oder organische Säure, wird der nicht umgesetzte β-Aminoalkohol aus dem Reaktionsgemisch durch Destillation, bevorzugt Kurzweg-, Dünnschicht- oder Fallfilmdestillation, entfernt. Flüssige β-Hydroxyalkylamide können ohne weiteren Reinigungsschritt verwendet werden. Feste β-Hydroxyalkylamide werden durch Kristallisation eventuell bei erhöhter Temperatur direkt aus dem Reaktionsgemisch oder aus einem geeigneten Lösemittel isoliert. Das Verfahren kann batchweise und/oder kontinuierlich durchgeführt werden.

Nach dem erfindungsgemäßen Verfahren können β-Hydroxyalkylamide nach Formel IV hergestellt werden: 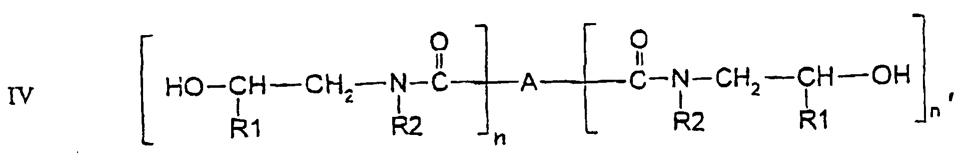 wobei A eine chemische Bindung oder eine mehrwertige organische Gruppe ist oder, wenn n'=0, kann A Wasserstoff oder eine einwertige organische Gruppe sein, wobei die ein- oder mehrwertige organische Gruppe ausgewählt ist aus gesättigten oder ungesättigten Alkylgruppen einschließlich substituierter Alkylgruppen, mit 1 bis 60 Kohlenstoffatomen, wie z.B. Methyl, Ethyl, Propyl, Butyl, Pentyl, Hexyl, Heptyl, Octyl, Nonyl, Decyl, Eicosyl, Triacontyl, Tetracontyl, Pentacontyl, Hexacontyl; Cycloalkylgruppen, wie z.B. Cyclopentyl, cyclohexyl; aromatische Kohlenwasserstoffgruppen, die einen oder mehrere Kerne enthalten, wie z.B. Phenyl, Naphthyl usw.; ungesättigte Gruppen, die eine oder mehrere ethylenische Gruppen (-C=C-) enthalten, wie z.B. Ethenyl, 1-Methylethenyl, 3-Butenyl-1,3-diyl, 2-Propenyl-1,2-diyl; Carboxynideralkenylgruppen, wie z.B. 3-Carboxy-2-propenyl usw.; Niederalkoxycarbonyl-Niederalkenylgruppen, wie z.B. 3-Methoxycarbonyl-2-propenyl usw., Niedertrialkylenaminogruppen, wie z.B. Trimethylenamino, Triethylenamino usw.. R1 ist Wasserstoff oder eine C1-C5-Akylgruppe, wie z.B. Methyl, Ethyl, n-Propyl, n-Butyl, sek-Butyl, tert.-Butyl, Pentyl usw. R2 ist Wasserstoff, eine C1-C5-Alkylgruppe oder: 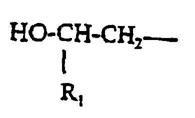 n ist eine ganze Zahl von 1 bis 10, bevorzugt 1 oder 2, und n' ist eine ganze Zahl von 0 bis 2.

Bevorzugt sind β-Hydroxyalkylamide der Formel IV, wobei A eine Alkylengruppe, bevorzugt C2 bis C₁₄, ist. Besonders bevorzugte β-Hydroxyalkylamide werden durch die vereinfachte Formel V wiedergegeben: 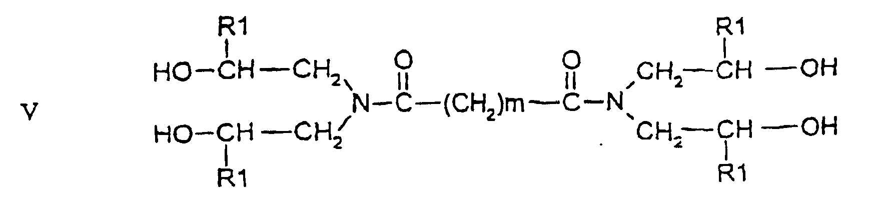 wobei m=2 bis 14 ist und R1 die bereits oben genannte Bedeutung hat.

Spezifische Beispiele für β-Hydroxyalkylamide nach Formel V sind N,N,N,'N'-Tetrakis(2-hydroxyethyl)-adipinsäureamid und N,N,N,'N'-Tetrakis(2-hydrox-propyl)-adipinsäureamid.

Die β-Hydroxyalkylamide (IV) werden erfindungsgemäß hergestellt ohne Lösemittel durch Aminolyse von Estern der Formel VI mit einem Überschuß der Amine der Formel VII bei geeigneten Temperaturen bis zu 200 °C in Gegenwart von basischen Katalysatoren. Die folgende Gleichung verdeutlicht den Prozeß:

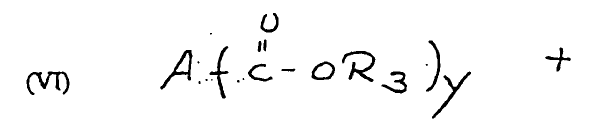

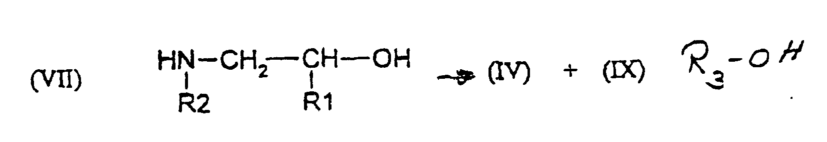

A, R1 und R2 haben dieselbe Bedeutung wie oben. Y=1 bis 20, R3 ist eine Alkylrest mit 1-5 Kohlenstoffatomen, wie z.B. Methyl, Ethyl, Propyl, n-Butyl, Tert.-butyl, Pentyl usw. Die Ester sind bekannte Produkte, die durch Veresterung der entsprechenden Säuren mittels Standardveresterungsverfahren, die dem Fachmann bekannt sind, hergestellt sind. Bevorzugte Säuren und Mischungen davon sind Oxalsäure, Malonsäure, Bernsteinsäure, Glutarsäure, Adipinsäure, Pimelinsäure, Korksäure, Azealinsäure, Sebazinsäure, Dodecandisäure, 1,4-Cyclohexandicarbonsäure und deren alkylsubstituierte Derivate usw. Verwendet werden können auch dimere und trimere Säuren und deren Mischungen, hergestellt durch Polymerisation von Fettsäuren, wie z.B. Dimersäure mit 2 Carboxylgruppen, 36 Kohlenstoffatomen und einem ungefähren Molekulargewicht von 565 oder Trimersäure mit 3 Carboxylgruppen, 54 Kohlenstoffatomen und einem ungefähren Molekulargewicht von 850.

Beispiele für erfindungsgemäße Amino der Formel VII sind 2-Aminoethanol, 2-Methylaminoethanol, 2-Ethylaminoethanol, 2-n-Propylaminoethanol, 2,2'-Iminodiethanol, 2-Aminopropanol, 2,2'-Iminodiisopropanol, 2-Aminocyclohexanol, 2-Aminocyclopentanol, 2-Aminomethyl-2-methylethanol 2-n-Butylaminoethanol, 2-Methylamino-1,2-dimethylethanol, 2-Amino-2-methyl-1-propanol, 2-Amino-2-methyl-1,3-propandiol, 2-Amino-2-ethyl-1,3-propandiol, 2-Amino-2-hydroxymethyl-1,3-propandiol und 1-Amino-2-propanol.

Die bei der Aminolyse (Reaktion von VI mit VII) entstehenden Alkohole der Formel IX werden aus dem Reaktionsgemisch durch Destillation, gegebenenfalls unter Vakuum aus dem Reaktionsgemisch entfernt. Aus Gründen der Selektivität ist ein molarer Überschuß an β-Aminoalkoholen notwendig, wobei das Verhältnis Esteräquivalent zu Aminäquivalent 1 : 1,001 bis 8, bevorzugt 1 : 1,05 bis 6, besonders bevorzugt 1 : 1,1 bis 1 : 2 beträgt. Durch diesen Überschuß werden Nebenreaktionen unterdrückt, wie z.B. die Bildung von nur teilweise aminierten Verbindung beim Einsatz von Estern von mehrbasigen Carbonsäuren, als Beispiel sei die Bildung von sogenannten Halbestern der Formel X beim Einsatz von Estern von mehrbasischen Carbonsäuren genannt. 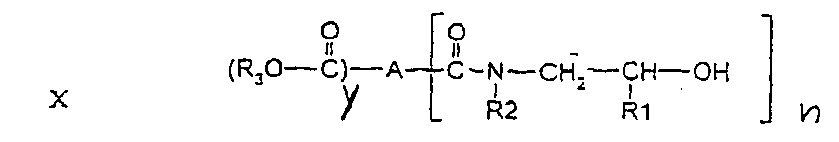 wobei A, R1, R2 und n die oben genannte Bedeutung haben und y=1 bis 5 ist.

Als weiteres Beispiel für Nebenprodukte, deren Bildung durch einen Überschuß von β-Aminoalkoholen unterdrückt werden können, sei die als Dimer bezeichnete Verbindung der Formel XI genannt, die als Nebenprodukt neben dem reinen monomeren β-Hydroxyalkylamiden der Formel IV auftritt. Monomer, Dimer und β-Aminoalkohol stehen im Gleichgewicht. 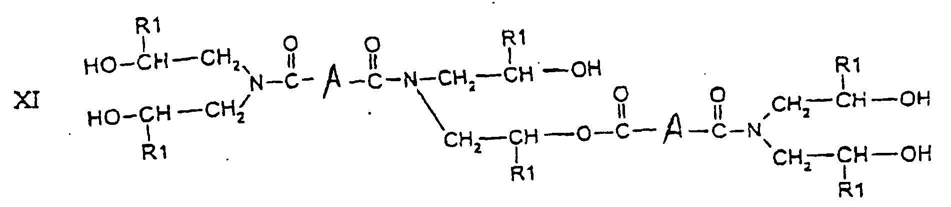 wobei A und R1 die oben genannte Bedeutung haben.

Wichtig zur Unterdrückung von Nebenreaktionen bei Aminolysereaktionen ist auch die Kontrolle des Wassergehaltes der Reaktionspartner. Typischerweise beträgt der Wassergehalt der Reaktionspartner weniger als 0,5 % bevorzugt weniger als 0,1 %, um die Hydrolyse der Ester und die Verringerung der Katalysatoraktivität zu verhindern.

Als Katalysatoren werden basische Katalysatoren vom Typ Alkalihydroxid oder -alkoholat, inklusive der quatärnären Ammoniumverbindungen, eingesetzt, wie z.B. Natriumhydroxid, Tetramethylammoniumhydroxid, Natriummethylat, Natrium-tert.butylat, Tertramethylammoniummethylat. Die eingesetzte Menge an Katalysatoren beträgt 0,001 bis 5,0 Mol.-%, bezogen auf das Gewicht der eingesetzten Ester.

Nachdem die Reaktion beendet ist, wird der Katalysator zerstört, z.B. durch Zugabe von anorganischen oder organischen Säuren, wie Salzsäure oder Essigsäure. Anschließend wird der überschüssige β-Aminoalkohol durch Destillation, gegebenenfalls unter Vakuum aus dem Reaktionsgemisch entfernt. Wird vor der Destillation der Katalysator nicht entfernt, so kommt es bei der Destillation wiederum zur Bildung von Nebenprodukten, z.B. bildet sich aus dem Monomer IV in Gegenwart von basischen Katalysatoren wiederum das Dimer XI. Diese Dimerbildung kann durch Zerstörung des Katalysators vor der Destillation unterdrückt werden. Bevorzugte Destillationsarten vor allem bei schwerflüchtigen β-Aminoalkoholen, die einen hohen Siedepunkt aufweisen, wie z.B. Diisopropanalamin, sind die Kurzweg-, Dünnfilm- oder Fallfilmdestillation, da bei diesen Arten der Destillation aufgrund der kurzfristigen Temperaturbelastung die β-Hydroxyalkylamide am wenigsten geschädigt werden. Ferner zeigte sich, daß der so abdestillierte β-Aminoalkohol aufgrund seiner hohen Reinheit ohne weitere Aufarbeitungsschritte wiederum als Ausgangskomponente für weitere Umsetzungen verwendet werden kann. Flüssige β-Hydroxyalkylamide können ohne weiteren Aufarbeitungsschritt weiterverarbeitet werden. Feste β-Hydroxyalkylamide werden durch Kristallisation eventuell bei erhöhter Temperatur direkt aus dem Reaktionsgemisch oder aus einem geeigneten Lösemittel isoliert. Bei der Kristallisation zeigt sich, daß je reiner die β-Hydroxyalkylamide sind, diese umso besser und schneller kristallisieren. Das Verfahren kann batchweise und/oder kontinuierlich durchgeführt werden.

Die Herstellung und die Eigenschaften der erfindungsgemäßen β-Hydroxyalkylamide werden nachfolgend beispielhaft dargestellt.

### Vergleichsbeispiel

Eine Mischung von 133,00 g Diisopropanolamin und 1,62 g Natriummethylat werden in einer 500 ml Glasapparatur unter Stickstoff auf 100 °C erhitzt. Nach Anlegen eines Vakuums von 300 mbar wird innerhalb einer Stunde 174,00 g Dimethyladipat zugetropft und das bei der Reaktion freiwerdende Methanol kontinuierlich abdestilliert. Nach einer Nachreaktionszeit von 1 Stunde wird das Produkt in eine Aluminiumschüssel abgelassen.

### Beispiel

Eine Mischung von 239,40 g Diisopropanolamin und 1,62 g Natriummethylat werden in einer 500 ml Glasapparatur unter Stickstoff auf 100°C erhitzt. Nach Anlegen eines Vakuums von 300 mbar wird innerhalb einer Stunde 174,00 g Dimethyladipat zugetropft und das bei der Reaktion freiwerdende Methanol kontinuierlich abdestilliert. Nach einer Nachreaktionszeit von 1 Stunde werden 1,80 g Essigsäure zum Reaktionsgemisch gegeben und anschließend wird das überschüssige Diisopropanolamin in der Kurzwegdestillationsapparatur KDL-5 der Fa. UIC bei einem Vakuum von 5 mbar und einer Manteltemperatur von 130°C entfernt. Das aminfreie Reaktionsgemisch wird anschließend bei 90 °C kristallisiert und danach wird der Kristallbrei in eine Aluminiumschüssel abgelassen.

**Tabelle 1**

| | Vergleichsbeipiel | Beispiel |
|---|---|---|
| Aspekt | Hochsviskose gelbe FlüsSigkeit. Nach wochenlangerLagerung wachsartige, klebrige Masse. | Freitfliessende weisse Kristalle |
| Schmelzpunkt [°C] | nicht bestimmbar | 118 |
| MonomergehaIt [%] | 73 | 97.9 |
| Dimergehalt [%] | 15 | 2.1 |
| Halbester [%] | 3 | - |
| Diisopropanolamin [%] | 9 | - |

## Patentansprüche

1. Verfahren zur Herstellung von β-Hydroxyalkylamiden der allgemeinen Formel IV: 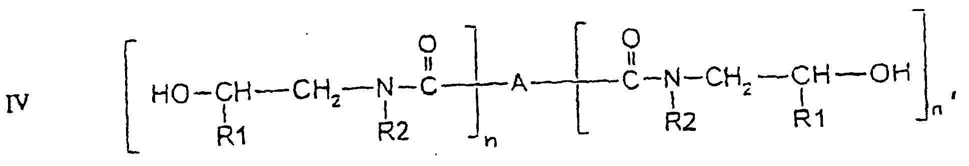 wobei A eine chemische Bindung oder eine mehrwertige organische Gruppe oder, wenn n'=0 Wasserstoff oder eine einwertige Gruppe ist, wobei die ein- oder mehrwertige organische Gruppe ausgewählt ist aus gesättigten oder ungesättigten (C1-C60) Alkyl-, Cycloalkyl-, Aryl-, Carboxyalkenyl-, Alkoxycarbonylalkenyl- oder Trialkylenaminogruppen und wobei R1 Wasserstoff oder eine C1-C5-Alkylgruppe und R2 Wasserstoff, eine C1-C5-Alkylgruppe oder: 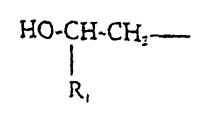 ist und wobei n eine ganze Zahl mit einem Wert von 1 bis 10 und n' eine ganze Zahl mit einem Wert von 0 bis 2 ist, bei dem in Abwesenheit von Lösemitteln die entsprechenden Alkylester mit den entsprechenden β-Aminoalkoholen in Gegenwart von basischem Katalysator umgesetzt und der entstehende Alkohol entfernt wird,
**dadurch gekennzeichnet,**
**daß** zur Verbesserung der Selektivität das Verhältnis Esteräquivalent zu Aminäquivalent 1:1,05 bis 6 beträgt, der basische Katalysator nach der Aminolyse zerstört und der nicht umgesetzte Aminoalkohol entfernt wird.

2. Verfahren nach Anspruch 1, **dadurch gekennzeichnet, daß** der nicht umgesetzte β-Aminoalkohol aus dem Reaktionsgemisch durch Destillation entfernt wird.

3. Verfahren nach einem der Ansprüche 1 bis 3, **dadurch gekennzeichnet, daß** zur Zerstörung des basischen Katalysators anorganische Säuren, bevorzugt Salzsäure oder organische Säuren, bevorzugt Essigsäure oder Phenylessigsäure verwendet werden.

4. Verfahren nach mindestens einem der Ansprüche 1 bis 3, **dadurch gekennzeichnet, daß** basische Katalysatoren vom Typ Alkalihydroxid oder -alkoholat, inklusive der quartanären Ammoniumverbindungen, eingesetzt werden, wie Natriumhydroxid, Tetramethylammoniumhydroxid, Natriummethylat, Natriumtert.butylat, Tertramethylammoniummethylat.

5. Verfahren nach einem der Ansprüche 1 bis 4, **dadurch gekennzeichnet, daß** die eingesetzte Menge an Katalysatoren 0,001 bis 5,0 Mol.-% bezogen auf das Gewicht der Ester, beträgt.

6. Verfahren nach mindestens einem der Ansprüche 1 bis 5, **dadurch gekennzeichnet, daß** der bei der Reaktion entstehende Alkohol bei einer Temperatur von 50-150 °C und einem Vakuum von 650 mbar bis 0,1 mbar entfernt wird.

7. Verfahren nach mindestens einem der Ansprüche 1 bis 6, **dadurch gekennzeichnet, daß** es kontinuierlich durchgeführt wird.

8. Verfahren nach einem der Ansprüche 1 bis 7, **dadurch gekennzeichnet, daß** bevorzugte β-Hydroxyalkylamiden nach Formel IV N,N,N,'N'-Tetrakis(2-hydroxyethyl)-adipinsäureamid und N,N,N,'N'-Tetrakis(2-hydroxpropyl)-adipinsäure-amid sind.

## Claims

1. Method for producingβ-hydroxyalkylamides with the general formula IV: 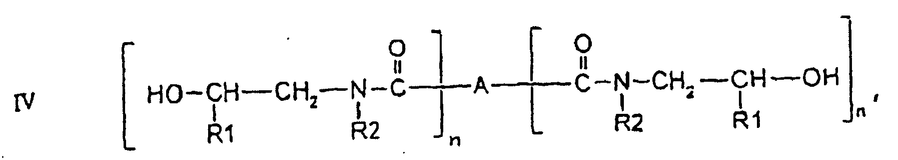 whereby A is a chemical connection or a multi-value organic group or, if n'=0 hydrogen or a single value group, whereby the single or multi-value organic group is chosen from saturated or unsaturated (C1-C60) alkyl-, cycloalkyl-, aryl-, carboxyalkenyl-, alkoxycarbonylalkenyl- or trialkylene amino groups, and whereby R1 is hydrogen or a C1-C5 alkyl group and R2 hydrogen, a C1-C5 alkyl group, or: 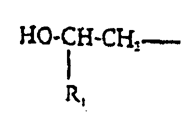 and whereby n is an integer with a value of 1 to 10, and n' is an integer with a value of 0 to 2, where in the absence of solvents the relevant alkylester is converted with the relevant β-aminoalcohols in the presence of a basic catalyst and the resulting alcohol removed, **characterised in that** for improving the selectivity of the ratio of ester equivalent to amino equivalent is 1:1, 0.5 to 6, the basic catalyst is destroyed following the aminolysis, and the non-converted amino alcohol removed.

2. Method according to Claim 1, **characterised in that** the non-converted β-aminoalcohol is removed from the reaction mixture by means of distillation.

3. Method according to one of the Claims 1 or 2 **characterised in that** anorganic acids, preferably hydrochloric acid or organic acids, preferably acetic acid or phenylacetic acid are used for the destruction of the basic catalyst.

4. Method according to one of the Claims 1 to 3, **characterised in that** the basic catalyst used is of the type alkalihydroxide or -alcoholate inclusive of the quatanary ammonium connections, such as sodium hydroxide, tetramethylammoniumhydroxide, sodium methylate, sodium tert.butylate, tertramethylammoniummethylate.

5. Method according to one of the Claims 1 to 4, **characterised in that** the quantity of catalyst used is 0.001 to 5.0 mol% of the weight of the ester.

6. Method according to at least one of the Claims 1 to 5, **characterised in that** the alcohol created during the reaction is removed at a temperature of 50-150°C and a vacuum of 650 mbar to 0.1 mbar.

7. Method according to at least one of the Claims 1 to 6, **characterised in that** the same is carried out continuously.

8. Method according to one of the Claims 1 to 7, **characterised in that** preferred β-hydroxyalkylamides according to formula IV are N,N,N,'N'-tetrakis (2-hydroxyethyl) - adipinacidamide and N,N,N,'N'-tetrakis (2-hydroxypropyl) -adipinacidamide.

## Revendications

1. Procédé de fabrication de ß-hydroxyalkylamides de formule générale IV : 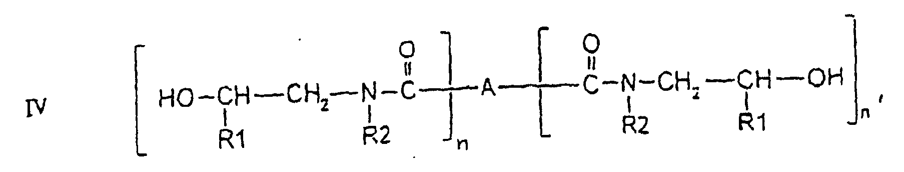 dans laquelle A est une liaison chimique ou un groupement organique polyvalent ou, si n'=0, A est l'hydrogène ou un groupement monovalent, le groupement organique mono- ou polyvalent étant choisi parmi des groupements saturés ou insaturés (en C₁-C₆₀) alkyle, cycloalkyle, aryle, carboxyalcényle, alcoxycarbonyl-alcényle ou trialkylèneamino, dans laquelle R1 est de l'hydrogène ou un groupement alkyle en C₁-C₅ et R2 de l'hydrogène, un groupement alkyle en C₁-C₅ ou : 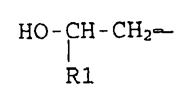 et dans laquelle n est un nombre entier d'une valeur de 1 à 10 et n' un nombre entier d'une valeur de 0 à 2, procédé dans lequel, en l'absence de solvants, on fait réagir les alkylesters correspondants avec les ß-aminoalcools correspondants en présence d'un catalyseur basique et on élimine l'alcool produit,
**caractérisé en ce que**, pour améliorer la sélectivité, le rapport de l'équivalent ester à l'équivalent amine est de 1:1,05 à 6, le catalyseur basique est détruit après l'aminolyse et l'aminoalcool qui n'a pas réagi est éliminé.

2. Procédé selon la revendication 1, **caractérisé en ce que** le ß-aminooalcool qui n'a pas réagi est éliminé du mélange réactionnel par distillation.

3. Procédé selon l'une quelconque des revendications 1 et 2, **caractérisé en ce que**, pour détruire le catalyseur basique, on utilise des acides inorganiques, de préférence de l'acide chlorhydrique, ou des acides organiques, de préférence de l'acide acétique ou de l'acide phénylacétique.

4. Procédé selon au moins l'une quelconque des revendications 1 à 3, **caractérisé en ce que** l'on utilise des catalyseurs basiques du type hydroxyde ou alcoolate d'alcali, notamment des composés d'ammonium quaternaire, tels que l'hydroxyde de sodium, l'hydroxyde de tétraméthylammonium, le méthylate de sodium, le tert-butylate de sodium et le méthylate de tétraméthylammonium.

5. Procédé selon l'une quelconque des revendications 1 à 4, **caractérisé en ce que** la quantité utilisée de catalyseurs est de 0,001 à 5,0% en mole par rapport au poids de l'ester.

6. Procédé selon au moins l'une quelconque des revendications 1 à 5, **caractérisé en ce que** l'alcool produit lors de la réaction est éliminé à une température de 50-150°C et sous un vide de 650 mbars à 0,1 mbar.

7. Procédé selon au moins l'une quelconque des revendications 1 à 6, **caractérisé en ce qu'**il est réalisé en continu.

8. Procédé selon l'une quelconque des revendications 1 à 7, **caractérisé en ce que** les β-hydroxyalkylamides préférés de formule IV sont l'amide de l'acide N,N,N',N'-tétrakis(2-hydroxyéthyl)-adipique et l'amide de l'acide N,N,N',N'-tétrakis(2-hydroxpropyl)-adipique.
